# EUROPÄISCHE PATENTSCHRIFT

(11) **EP 1 658 486 B1**
(45) Veröffentlichungstag und Bekanntmachung des Hinweises auf die Patenterteilung: **10.09.2008**
(21) Anmeldenummer: 04763290.6
(22) Anmeldetag: 16.07.2004
(51) Int. Cl.: G01N 21/25

(54) **VERFAHREN ZUR SPEKTROSKOPISCHEN MESSUNG AN PARTIKELPROBEN UND MESSEINRICHTUNG ZUR DURCHFÜRHRUNG DES VERFAHRENS**
METHOD FOR PERFORMING A SPECTROSCOPIC MEASUREMENT ON PARTICLE SAMPLES AND MEASURING DEVICE FOR CARRYING OUT SAID METHOD
PROCEDE POUR EFFECTUER UNE MESURE SPECTROSCOPIQUE SUR DES ECHANTILLONS PARTICULAIRES ET DISPOSITIFS DE MESURE POUR LA MISE EN OEUVRE DUDIT PROCEDE

(30) Priorität: 18.07.2003 DE 10332800
(43) Veröffentlichungstag der Anmeldung: 24.05.2006
(73) Patentinhaber: Bruins, Hans Joachim, 81247 München (DE)
(72) Erfinder: Bruins, Hans Joachim, 81247 München (DE)
(74) Vertreter: Hertz, Oliver
(86) Internationale Anmeldenummer: PCT/EP2004/007955
(87) Internationale Veröffentlichungsnummer: WO 2005/010505

(56) Entgegenhaltungen:
- WO-A-01/09590
- DE-A- 3 413 065
- US-A- 4 692 620
- US-A- 5 448 069
- US-A1- 2003 063 276
- US-B1- 6 278 518
- PATENT ABSTRACTS OF JAPAN Bd. 2000, Nr. 02, 29. Februar 2000 (2000-02-29) & JP 11 304699 A (KETT ELECTRIC LAB), 5. November 1999 (1999-11-05)
- PATENT ABSTRACTS OF JAPAN Bd. 2003, Nr. 10, 8. Oktober 2003 (2003-10-08) & JP 2003 161697 A (ISEKI & CO LTD), 6. Juni 2003 (2003-06-06)

## Beschreibung

Die Erfindung betrifft ein Messverfahren mit den Merkmalen des Oberbegriffs von Anspruch 1, eine zur Durchführung eines derartigen Messverfahrens vorgesehene Messeinrichtung und eine Getreidefördereinrichtung, die mit dieser Messeinrichtung ausgestattet ist.

Aus DE 101 197 63 A1 ist eine Getreideanalysetechnik bekannt, deren Funktionsprinzip in Figur 4 schematisch illustriert ist. Zur Messung spektroskopischer Eigenschaften von Getreide 50' wird dieses mit einem Schaufelrad 12' in einen Fallschacht 20' transportiert und dort an einer Messstrecke 21' (Lichtweg zwischen Lichtquelle und Detektor) einer Erfassung von spektroskopischen Parametern des Getreides 50' unterzogen. Am Fallschacht 20' ist eine der Messstrecke 21' nachgeordnete Sperreinrichtung 23' vorgesehen, die derart betätigt wird, dass das Getreide im Fallschacht 20' abwechselnd bewegt wird oder ruht, wobei die spektroskopische Messung am ruhenden Getreide 50' erfolgt.

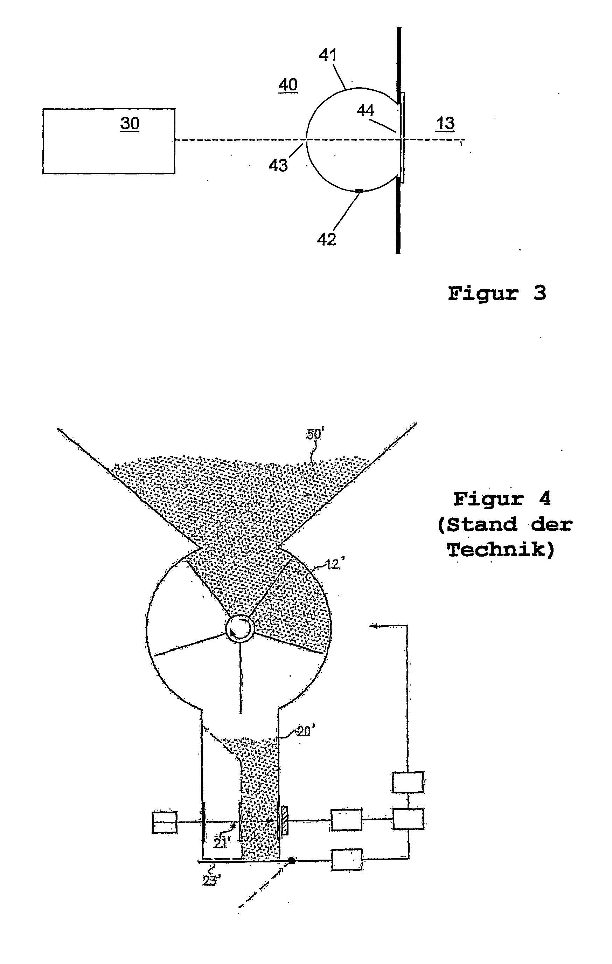

Die herkömmliche Technik gemäß Figur 4 kann bei bestimmten Anwendungen nachteilig sein, da der Aufbau mit dem Schaufelrad 12', dem Fallschacht 20' und der Sperreinrichtung 23' relativ komplex ist, einen großen Herstellungsaufwand erfordert und unter praktischen Anforderungen störanfällig sein kann.

Aus der Praxis sind auch Getreidemesseinrichtungen für den mobilen Einsatz bekannt, bei denen das Beleuchtungs- und Messlicht über Lichtleiter zur Probe übertragen wird. Diese Technik ist nachteilig, da die Übertragungseigenschaften der Lichtleiter empfindlich durch äußere mechanische Einflüsse, wie z.B. Vibrationen oder Verspannungen gestört werden können, so dass mit dieser Technik nur mit beschränkter Genauigkeit gemessen werden kann.

Die genannten Nachteile herkömmlicher Getreideanalysetechniken sind auch bei der Messung an anderen Proben in Form von Partikeln, wie z.B. Kunststoffgranulaten in der Kunststoffindustrie gegeben.

Die JP 11304699 offenbart eine Messeinrichtung zur Analyse von Körnern, wie beispielsweise Reis, mit den Merkmalen der Oberbegriffe der unabhängigen Ansprüche.

Die Aufgabe der Erfindung ist es, verbesserte Verfahren zur Messung spektroskopischer Eigenschaften von Proben, die eine Vielzahl von Partikeln aufweisen, bereitzustellen, die die Nachteile der herkömmlichen Techniken vermeiden und insbesondere einen vereinfachten Messaufbau bei gleichbleibender oder verbesserter Genauigkeit und Reproduzierbarkeit der Messung ermöglichen. Die Aufgabe der Erfindung ist es auch, verbesserte Messeinrichtungen zur Durchführung derartiger Messverfahren bereitzustellen, die sich insbesondere durch einen vereinfachten Aufbau, eine erhöhte Genauigkeit und eine erhöhte Betriebszuverlässigkeit auszeichnen. Eine weitere Aufgabe der Erfindung ist es, Anwendungen derartiger Messeinrichtungen anzugeben.

Diese Aufgaben werden durch Verfahren, Messeinrichtungen und Getreidefördereinrichtungen mit den Merkmalen der unabhängigen Patentansprüche gelöst. Vorteilhafte Ausführungsformen und Anwendungen der Erfindung ergeben sich aus den abhängigen Ansprüchen.

Verfahrensbezogen wird die oben genannte Aufgabe durch die allgemeine technische Lehre gelöst, zur spektroskopischen Messung den mindestens einen spektroskopischen Parameter der Probe zu erfassen, während diese sich in einem Transportrad befindet. Als Transportrad ist allgemein eine rotierende Fördereinrichtung (Förderrad) mit mindestens einer Transportkammer für die partikelförmige, schütt- und rieselfähige Probe vorgesehen, wobei sich die Probe während der Förderung im freien Raum der mindestens einen Transportkammer befindet. Erfindungsgemäß trifft ein von einer Beleuchtungseinrichtung ausgehender Lichtweg auf die Probe in der Transportkammer. Der Lichtweg von der Beleuchtungseinrichtung zu einer Detektoreinrichtung bildet eine optische Messstrecke, die zu der oder gegebenenfalls durch die Transportkammer führt. Die Messung kann erfolgen, während die Probe gefördert wird. Die erfindungsgemäße Messung an der Probe im Transportrad besitzt den Vorteil, dass der Messaufbau im Vergleich zur herkömmlichen Technik erheblich vereinfacht wird. Des Weiteren hat sich gezeigt, dass die Messung insbesondere an Getreide mit einer hohen Genauigkeit und Reproduzierbarkeit möglich ist.

Gemäß der Erfindung wird - das Transportrad diskontinuierlich betrieben. Dies bedeutet, dass sich gemäß einer ersten Variante Förderbewegungen (Rotationsbewegungen) des Transportrades mit verschiedenen Richtungen abwechseln. Dabei kann vorteilhafterweise durch die bei der Richtungsumkehr erzielte Umordnung der Probenpartikel eine Verdichtung der Probe in der Transportkammer erreicht werden, so dass sich die Messgenauigkeit und Reproduzierbarkeit der Messung weiter erhöhen kann. Gemäß einer zweiten Va-riante der Offenbarung sind abwechselnd mindestens eine Rotationsbewegung und ein Ruhezustand des Transportrades vorgesehen, wobei im Ruhezustand vorteilhafterweise die Probe unbeweglich ist. Beide Varianten können aufeinanderfolgend realisiert werden, indem zunächst eine Verdichtung durch Rotationsbewegungen mit Richtungswechsel erfolgen und anschließend der Ruhezustand eingenommen wird. Gemäß einer besonders bevorzugten Ausführungsform der Erfindung erfolgt die spektroskopische Messung während sich das Transportrad nicht bewegt und die Probe relativ zur optischen Messstrecke ruht.

Wenn gemäß einer vorteilhaften Variante das Transportrad mehrere Transportkammern aufweist, die jeweils Teilproben aufnehmen können, ist vorzugsweise vorgesehen, dass die Probe, die sich im Lichtweg befindet, durch benachbarte, Teilproben und/oder durch benachbarte Transportkammern gegenüber unerwünschtem Fremdlicht abgeschirmt wird. Durch diese Abschir-mung können vorteilhafterweise Fehlmessungen vermieden werden.

Erfindungsgemäß kann an der Probe im Transportrad jede an sich bekannte spektroskopische Messung, d.h. die Messung mindestens eines spektroskopischen Parameters durchgeführt werden, der für die Wechselwirkung der Probe mit Licht (IR-Strahlung, sichtbare Strahlung oder UV-Strahlung) charakteristisch ist. Besonders bevorzugt werden die Transmission, die optische Dichte und/oder die Reflexion der Probe erfasst. Diese Messgrößen besitzen den Vorteil, dass sie insbesondere bei der Messung an Getreide eine hohe Aussagekraft über physikalische oder chemische Eigenschaften der Getreidekörner besitzen und die Messung mit an sich verfügbaren Auswertungs-oder Datenverarbeitungstechniken kompatibel ist. Die Reflexionsmessung besitzt den zusätzlichen Vorteil, dass ein kompakter Messaufbau ermöglicht wird, bei dem die Beleuchtungseinrichtung und die Detektoreinrichtung auf einer Seite des Transportrades angeordnet sind. Besonders bevorzugt ist eine Differenzwert- und/oder -spektrenbildung vorgesehen, die insbesondere bei der Messung an Getreide in kurzer Zeit eine große auswertbare Informationsmenge liefert.

Ein Kammergehäuse, in dem sich das Transportrad befindet, bildet mit den Förderflächen des Transportrades eine Messkammer (Messküvette), die sich im Lichtweg der Beleuchtungseinrichtung befindet. Vorteilhafterweise kann die Länge der durch die Messkammer verlaufenden optischen Messstrecke, also die Schichtdicke der Probe in der Messstrecke durch eine Erweiterung oder Verkleinerung des Kammergehäuses und/oder des Transportrads eingestellt werden. Durch diese Gestaltung kann der Messaufbau vorteilhafterweise mit geringem Aufwand an die Absorptions- oder Reflexionseigenschaften der vermessenen Probe angepasst werden.

Wenn gemäß einer bevorzugten Ausführungsform der Erfindung die optische Messstrecke parallel zur Rotationsachse des Transportrades verläuft, kann dadurch vorteilhafterweise ein besonders kompakter Messaufbau realisiert werden.

Das Messverfahren ist allgemein an Proben aus synthetischen Partikeln, insbesondere Kunststoffpartikeln oder -granulaten, oder an partikelförmigen Naturprodukten, insbesondere landwirtschaftlichen Produkten in Form von Samen- oder Fruchtkörpern anwendbar. Typische Partikelgrößen liegen im Bereich von 0,1 mm bis 20 mm. Besonders bevorzugt ist die Messung an Getreide, Mais, Soja oder dgl.

Vorrichtungsbezogen wird die oben genannte Aufgabe durch eine Messeinrichtung zur Messung spektroskopischer Eigenschaften einer partikelförmigen Probe dahingehend gelöst, dass eine optische Messstrecke der Messeinrichtung durch ein Transportrad zur Förderung der Probe verläuft oder auf das Transportrad gerichtet ist. Die Messeinrichtung ist vorteilhafterweise wesentlich einfacher und weniger störanfällig aufgebaut als die herkömmlichen Messeinrichtungen.

Gemäß einer bevorzugter Ausführungsform der Erfindung wird als Transportrad ein Schaufelrad in einem Kammergehäuse verwendet, wobei die optische Messstrecke zumindest einseitig durch das Kammergehäuse verläuft. Die Verwendung eines Schaufelrades besitzt den Vorteil, dass Schaufelräder an sich in der Fördertechnik, insbesondere in der Getreidefördertechnik, verfügbar sind und üblicherweise angewendet werden. Gemäß abgewandelten Varianten kann als Transportrad entsprechend eine andere Fördereinrichtung, wie zum Beispiel ein Schneckenförderer vorgesehen sein.

Das Kammergehäuse ist vorzugsweise an mindestens einer Wand, in der Regel an zwei gegenüberliegenden Wänden jeweils mit einem transparenten Fenster für die optische Messstrecke ausgestattet. Im übrigen kann das Kammergehäuse aus einem nichttransparenten Material hergestellt sein, so dass vorteilhafterweise die optische Abschirmung der Probe gegenüber Fremdlicht im Schaufelrad verbessert wird.

Das transparente Fenster im Kammergehäuse wird durch einen optischen Filter gebildet, wobei vorteilhafterweise eine Anpassung des Beleuchtungs- und/oder Messlichts entlang der Messstrecke im Transportrad an die jeweilige spektroskopische Messung und/oder eine Abschirmung von Fremdlicht (z. B. Tageslicht) erfolgen können. Besondere Vorteile für einen sicheren und reibungslosen Messbetrieb ergeben sich, wenn der Filter oder allgemein das Fenster in der Wand des Kammergehäuses aus einem Material gebildet ist, das unempfindlich gegen elektrostatische Aufladungen ist. Dadurch kann ein Anhaften von Probenpartikeln oder Verunreinigungen am Fenster vermieden werden.

Ein weiterer Gegenstand der Erfindung ist eine Getreidefördereinrichtung, die mit der erfindungsgemäßen Messeinrichtung ausgestattet ist und eine Zuführeinrichtung, die Messeinrichtung und eine Auffangeinrichtung für Getreide aufweist.

Gemäß einer vorteilhaften Ausführungsform der Erfindung kann die Getreidefördereiririchtung in eine Getreide-Transportleitung integriert werden, indem die Zuführeinrichtung beispielsweise als Abzweigung an eine Rohrleitung angekoppelt wird, durch die Getreide z.B. in einem Getreidelager oder einem Verarbeitungsbetrieb bewegt wird.

Weitere Vorteile und Einzelheiten der Erfindung werden aus der folgenden Beschreibung von bevorzugten Ausführungsformen ersichtlich. Es zeigen:
- Fig. 1: eine schematische Illustration einer Ausführungsform der erfindungsgemäßen Messeinrichtung;
- Fig. 2: eine Perspektivansicht einer Ausführungsform der erfindungsgemäßen Messseinrichtung;
- Fig. 3: eine schematische Illustration einer erfindungsgemäß verwendeten Ablenkeinrichtung zur Sammlung diffus reflektierten Lichtes; und
- Fig. 4: eine schematische Schnittdarstellung einer herkömmlichen Messeinrichtung (Stand der Technik).

Eine erfindungsgemäße Messeinrichtung 100 umfasst als Transportrad ein Schaufelrad 10, eine Beleuchtungseinrichtung 30 und eine Detektoreinrichtung 40. Ein Lichtweg 34, der die optische Messstrecke bildet (gestrichelt eingezeichnet) verläuft zwischen der Beleuchtungseinrichtung 30 und der Detektoreinrichtung 40.

Das Schaufelrad 10 umfasst fünf Flügel 11, die entsprechend fünf Transportkammern 13 bilden. Die Flügel 11 sind ebene Platten, die von einem zentralen Träger 12 radial abstehen. Die Rotationsachse des Schaufelrades 12 verläuft parallel zur Ausrichtung des Lichtwegs 34. Die Rotation des Schaufelrads 12 wird mit einer Stelleinrichtung 27 (ausschnittsweise gezeigt) aktiviert. Die Stelleinrichtung 27 ist mit einer Lochscheibe 28 ausgestattet, die sich mit dem Schaufelrad 10 mitdreht und der Positionierung des Schaufelrades 10 dient.

Eine der Transportkammern 13 kann durch eine Verbindung zwischen den freien Enden der angrenzenden Flügel verschlossen sein, so dass sie während des Betriebs leer bleibt. An dieser leeren Transportkammer können Referenzmessungen durchgeführt werden.

Das Schaufelrad 10 ist in einem Kammergehäuse 20 angeordnet. Das Kammergehäuse 20 besteht aus zwei schalenförmigen Basis-und Deckelteilen 21, 22. Das Basisteil 21 umfasst eine Grundplatte, die eine seitliche Wand des Kammergehäuses 20 bildet und in der das Schaufelrad drehbar gelagert ist, und einen von der Grundplatte abstehenden, umlaufenden Rand. Das Deckelteil 22 umfasst eine Deckplatte, die eine weitere seitliche Wand des Kammergehäuses 20 bildet, und einen von der Deckplatte abstehenden, umlaufenden Rand. Das Kammergehäuse 20 mit dem Schaufelrad 10 ist senkrecht so ausgerichtet, dass die Drehachse des Schaufelrades 10 horizontal verläuft.

Die Beleuchtungseinrichtung 30 umfasst eine Messlichtquelle (nicht gezeigt) und ggf. eine Einrichtung zur Bereitstellung von Messlicht mit einer vorbestimmten, gegebenenfalls durchstimmbaren spektralen Wellenlängenverteilung. Beispielsweise sind eine Weißlichtquelle und ein Monochromator 31 (oder optischer Filter) vorgesehen. Alternativ können mindestens eine Leuchtdiode oder mindestens eine Laser-Lichtquelle (z.B. Laserdiode) vorgesehen sein. Des Weiteren kann die Messlichtquelle eine Optik zur Strahlformung enthalten, so dass das Messlicht entlang des Lichtwegs 34 einen im wesentlichen parallelen Strahlengang besitzt.

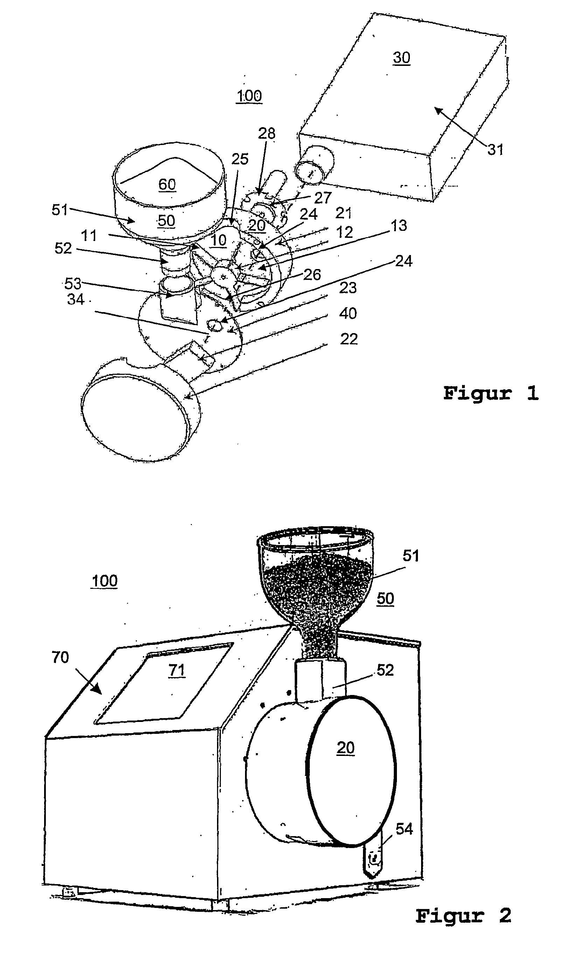

Die Detektoreinrichtüng 40 enthält einen an sich bekannten lichtempfindlichen Sensor, wie z.B. eine Photodiode, der mit einem Verstärker und einer Steuereinrichtung (nicht dargestellt) verbunden ist. Die Detektoreinrichtung 40 ist bei dem in Figur 1 illustrieiten, für Transmissions- oder Dichtemessungen vorgesehenen Ausführungsbeispiel der erfindungsgemässen Messeinrichtung am Deckelteil 22 angeordnet. Zum Schutz der Detektoreinrichtung 40 und/oder als Träger für Fenster und/oder einen Filter ist zwischen dem Schaufelrad 10 und der Detektoreinrichtung 40 eine plattenförmigen Trennwand 23 vorgesehen. Ist die Messeinrichtung hingegen für eine Reflexionsmessung vorgesehen, wird die Detektoreinrichtung auf der selben Seite des Kammergehäuses 20 wie die Beleuchtungseinrichtung angeordnet (siehe unten).

In der Grundplatte des Basisteils 21 und der Trennwand 23 sind zueinander ausgerichtet zwei Fenster vorgesehen, durch die die optische Messstrecke (Lichtweg 34) verläuft. Die Fenster 24 sind beispielsweise mit Glas, insbesondere Filterglas mit einem Kantenfilter verschlossen. Die optischen Filter bestehen vorzugsweise aus einem Material, das unempfindlich gegen elektrostatische Aufladungen ist. Für IR-Transmissionsmessungen werden vorzugsweise Rot-Grün-Filter (715 nm-Kantenfilter) aus FeO verwendet. Dies ermöglicht eine Abschirmung von Tageslicht, so dass die Messeinrichtung bei Tageslicht betrieben werden. Ein weiterer, insbesondere für die Messung an Getreide wichtiger Vorteil bei der Verwendung von FeO-Filtern besteht darin, dass diese eine Härte besitzen, die ein Zerkratzen der Filteroberfläche durch die mit dem Schaufelrad bewegte Probe verhindert. Durch die erfindungsgemäße Verwendung von FeO-Filtermaterial wird die Funktionsfähigkeit und Reproduzierbarkeit der spektroskopischen Messungen auch bei langen Betriebszeiten erhalten.

Die Ränder der Basis- und Deckelteile 21, 22 bilden an ihren oberen und unteren Seiten jeweils Öffnung 25, die der Zufuhr und Abfuhr der Probe aus dem Kammergehäuse bei der Transmissions- oder Dichtemessung dienen. Die obere Öffnung 25 wird durch bogenförmige Ausnehmungen an den angrenzenden Rändern der Basis- und Deckelteile 21, 22 gebildet und dient der Aufnahme eines Adapters 53 einer Zufuhreinrichtung 50.

Die Zufuhreinrichtung 50 wird beim dargestellten Ausführungsbeispiel durch einen Trichter 51, ein Verbindungsstück 52 und den Adapter 53 gebildet. Der Adapter 53 ist zur Einführung der Probe durch die Öffnung 25 in die oberste Transportkammer des Schaufelrades 10 eingerichtet. Zur Lichtabschirmung ist die Außenform der Öffnung 25 an die Form des Adapters 53 angepasst. Es wird betont, dass in Figur 1 der Adapter lediglich zu Illustrationszwecken zwischen der Trennwand 23 und dem Deckelteil 23 gezeigt ist. Bei bestimmungsgemäßer Zusammensetzung der erfindungsgemäßen Messeinrichtung ist der Adapter 53 so an das Basisteil 21 angrenzend angeordnet, dass die Probe 60 in die oberste Transportkammer gefüllt werden kann. Hierzu kann in der Trennwand 23 eine Aufnahme zur Positionierung des Adapters 53 vorgesehen sein. Alternativ kann als Zuführeinrichtung ein Anschlussstück (z.B. T-Verbindungsstück) zur Kopplung der Messeinrichtung an eine Rohrleitung, aus der Teilproben entnommen werden sollen, vorgesehen sein.

Der zwischen den Fenstern 24 gebildete Teilabschnitt bildet die optische Messstrecke des Lichtweges 34 beispielsweise mit einer Länge im Bereich von 18 mm bis 30 mm. Diese Länge legt bei der Transmissions- oder Dichtemessung die Schichtdicke der spektroskopischen Messung fest. Die Schichtdicke kann an das jeweils vermessene Material durch Auswahl eines Schaufelrades mit einer geeigneten Flügelbreite und durch Einlegen von Abstandshaltern (scheibenförmige Platten mit Öffnungen entsprechend der Position der Fenster) in das Kammergehäuse 20 angepasst werden. Das Volumen einer Transportkammer des Schaufelrads 12 umfasst je nach Anwendungsfall beispielsweise 50 ml bis 150 ml, wobei alternativ kleinere oder größere Kammervolumen vorgesehen sein können.

Zur Durchführung einer erfindungsgemäßen Messung wird die Probe 60 (nur im Trichter 51 gezeigt) zunächst in die oberste Transportkammer des Schaufelrades 10 und bei dessen Betätigung in die optische Messstrecke (Lichtweg 34) bewegt. Vor der Messung erfolgen Rüttel- oder Rotationsbewegungen mit abwechselnder Rotationsrichtung des Schaufelrades 10, so dass sich die Probe in der Transportkammer 13 verdichtet. Vorteilhafterweise wird das Getreide in der Transportkammer, durch die die Messstrecke verläuft, durch Getreide in benachbarten Transportkammern und die Flügel des Schaufelrades gegen störendes Fremdlicht abgeschirmt.

Anschließend erfolgt die spektroskopische Messung, z.B. die Aufnahme eines Transmissionsspektrums. Nach der Aufnahme des Spektrums wird das Schaufelrad um 1/5 seines Umfangs weiter gedreht, so dass eine weitere Teilprobe in die optische Messstrecke 34 geführt wird. Die Anzahl der Messungen an Teilproben wird je nach Art der Probe und den Anwendungsbedingungen gewählt. Die Dauer der Messung einer Teilprobe beträgt vorteilhafterweise nur 2 bis 3 s. Für die Messung an Getreide erfolgen vorzugsweise Messungen an rund zehn bis sechzehn Teilproben. Bei der weiteren Rotation des Schaufelrades 12 fallen Teilproben, die bereits gemessen wurden, an der unteren Öffnung 26 in eine Auffangeinrichtung (z. B. ein Auffangbehälter, nicht gezeigt).

Für die Anwendung in der Praxis ist die erfindungsgemäße Messeinrichtung 100 entsprechend der in Figur 2 illustrierten Ausführungsform mit einem Hauptgehäuse 70 ausgestattet, in dem die Beleuchtungseinrichtung, die Detektoreinrichtung (Reflexionsmessung), die Stelleinrichtung zur Betätigung des Schaufelrades, eine Steuer- und Auswertungseinrichtung und eine Bedien- und Anzeigeeinrichtung 71 angeordnet sind. An der Außenseite des Hauptgehäuses 70 ist das Kammergehäuse 20 mit dem Schaufelrad angeordnet. An der Oberseite des Kammergehäuses 20 befindet sich das Verbindungsstück 52 zur Aufnahme des Trichters 51 der Zufuhreinrichtung 50. Alternativ kann an dieser Stelle eine Verbindung mit einer Getreidefördereinrichtung oder einer Getreide-Transportleitung vorgesehen sein. Das Bezugszeichen 54 bezeichnet einen Fallschacht, durch den die gemessene Probe in eine Auffangeinrichtung fällt.

Figur 2 illustriert die vorteilhafte, kompakte Bauform der erfindungsgemäßen Messeinrichtung, wenn diese für die Reflexionsmessung vorgesehen ist. In diesem Fall kann das Kammergehäuse 20 relativ flach gebildet werden, da es lediglich das Schaufelrad und keinen Detektor enthält. Des Weiteren werden zusätzliche elektrische Leitungen vom Kammergehäuse zur Steuereinrichtung im Hauptgehäuse 70 vermieden. Die Detektoreinrichtung (nicht dargestellt), befindet sich im Hauptgehäuse 70 neben der Beleuchtungseinrichtung.

Bei Reflexionsmessungen verläuft der Lichtweg 34 von der Beleuchtungseinrichtung auf die Probe in der Transportkammer des Schaufelrades und zurück zur Detektoreinrichtung. Diese ist mit einer Ablenkeinrichtung ausgestattet, mit der das reflektierte Licht zu einem außerhalb des Verlaufs des Lichtwegs 34 von der Beleuchtungseinrichtung zur Probe, außeraxial angeordneten lichtempfindlichen Sensor abgelenkt wird. Da das Messlicht von der partikelförmigen Probe in der Transportkammer diffus rückgestreut wird, ist als Ablenkeinrichtung vorteilhafterweise eine Integrationskugel vorgesehen, die mit weiteren Einzelheiten in Figur 3 schematisch gezeigt ist.

Gemäß Figur 3 verläuft der Lichtweg 34 zunächst von der Beleuchtungseinrichtung 30 zur Transportkammer 13 mit der partikelförmigen Probe. Von dort wird das Messlicht diffus zurück reflektiert. Die Detektoreinrichtung 40 umfasst die Integrationskugel 41 und einen lichtempfindlichen Sensor 42, wie zum Beispiel eine Photodiode. Die Integrationskugel 41 besitzt auf der Seite der Beleuchturigseinrichtung 33 eine kleine Öffnung 43, durch die der Lichtweg 34 verläuft. Diese Öffnung 43 besitzt einen geringen Durchmesser von lediglich wenigen Millimetern. Auf der entgegen gesetzten Seite besitzt die Integrationskugel 41 eine Öffnung mit einem größeren Durchmesser, der den Durchmesser des Fensters 24 in der angrenzenden Basisplatte 21 (siehe Figur 1) entspricht. Die Öffnung 44 wird gebildet, in dem die Integrationskugel 41 einseitig angeschnitten ist. Die Integrationskugel 41 besitzt einen Durchmesser von z.B. 3 mm bis 4 mm.

Die Innenseite der Integrationskugel 41 ist mit einer hoch reflektierenden Beschichtung (z. B. aus Gold) versehen. Der Sensor 42 ist seitlich in die Oberfläche der Integrationskugel 41, z. B. mit einem Winkel von 90 Grad gegenüber dem Lichtweg 34 eingebettet.

Die als Ablenkeinrichtung wirkende Integrationskugel 41 besitzt den Vorteil, dass das diffus reflektierte Licht mit einer großen Effektivität am Sensor 42 gesammelt werden kann.

Die erfindungsgemäße Messtechnik besitzt die folgenden wichtigen Vorteile. Der Aufbau ist mechanisch robust. Die Funktionssicherheit ist erhöht, da nur noch das Schaufelrad ein bewegliches Teil darstellt. Die Messung kann mit einer hohen Genauigkeit erfolgen. Es wurde eine Standardabweichung von 0.05 erreicht, wohingegen die Standardabweichung bei herkömmlichen Techniken 0.8 beträgt. Entsprechend kann, um gleich bleibend gute Ergebnisse zu erzielen, mit einer kleiner dimensionierten Beleuchtungseinrichtung gearbeitet werden, was insbesondere für den mobile Einsatz von Vorteil ist. Die Messung kann mit einer hohen Geschwindigkeit erfolgen, wie es zum Beispiel bei online-Anwendungen in Getreideannahmestellen, beim Handel, in Lagern oder bei Getreidemischern erforderlich ist.

Die Erfindung ist nicht auf das vorstehend beschriebene bevorzugte Ausführungsbeispiel beschränkt. Vielmehr ist eine Vielzahl von Varianten und Abwandlungen möglich, die ebenfalls von der Erfindung Gebrauch machen und deshalb in den Schutzbereich der Ansprüche fallen.

## Patentansprüche

1. Verfahren zur Messung von mindestens einem spektroskopischen Parameter einer Probe (60), die eine Vielzahl von Partikeln umfasst,
- bei dem die Probe (60) mit einem Transportrad (10) in einen Lichtweg (34) einer Beleuchtungseinrichtung (30) transportiert und
- dort der Messung des mindestens einen spektroskopischen Parameters unterzogen wird, wobei
- die Messung des mindestens einen spektroskopischen Parameters erfolgt, während sich die Probe (60) in einer Transportkammer (13) des Transportrads (10) befindet,
- der Lichtweg (34) auf die Transportkammer (13) gerichtet ist,
- das Transportrad (10) in einem Kammergehäuse (20) angeordnet ist, und wobei
- in mindestens einer Wand des Kammergehäuses (20) ein transparentes Fenster (24) vorgesehen ist, durch das der Lichtweg (34) verläuft,
**dadurch gekennzeichnet, dass**
- das transparente Fenster (24) durch einen optischen Filter gebildet wird und
- das Transportrad (10) diskontinuierlich betrieben wird, wobei sich Förderbewegungen des Transportrads (10) mit verschiedenen Richtungen abwechseln, so dass die Probe (60) in der Transportkammer (13) verdichtet wird.

2. Verfahren nach Anspruch 1, bei dem die Erfassung des mindestens einen spektroskopischen Parameters erfolgt, während sich das Transportrad (10) nicht bewegt und die Probe (50) relativ zum Lichtweg (34) ruht.

3. Verfahren nach Anspruch 2, bei dem vor der Erfassung des mindestens einen spektroskopischen Parameters eine Verdichtung der Probe (60) erfolgt.

4. Verfahren nach mindestens einem der vorhergehenden Ansprüche, bei dem als spektroskopischer Parameter die Transmission, die optische Dichte und/oder die Reflexion der in der Messstrecke (34) befindlichen Probe (50) erfasst wird.

5. Verfahren nach Anspruch 4, bei dem eine Differenzwertund/oder -spektrenbildung erfolgt.

6. Verfahren nach mindestens einem der vorhergehenden Ansprüche, bei dem eine Schichtdicke der Probe (60) entlang des Lichtwegs (34) durch eine Erweiterung oder Verkleinerung der Transportkammer (13) eingestellt wird.

7. Verfahren nach mindestens einem der vorhergehenden Ansprüche, bei dem als Transportrad ein Schaufelrad (10) mit einer oder mehreren Transportkammern (13) verwendet wird.

8. Verfahren nach mindestens einem der vorhergehenden Ansprüche, bei dem der Lichtweg (34) parallel zur Rotationsachse des Schaufelrads (12) verläuft.

9. Verfahren nach mindestens einem der vorhergehenden Ansprüche, bei dem die Probe (60) partikelförmige landwirtschaftliche Produkte oder synthetische Partikel umfasst.

10. Verfahren nach Anspruch 9, bei dem die Probe (60) Getreide umfasst.

11. Messeinrichtung (100) zur Messung spektroskopischer Eigenschaften einer Probe (60) mit einer Vielzahl von Partikeln, umfassend:
- eine Beleuchtungseinrichtung (30) und eine Detektoreinrichtung (40), zwischen denen ein Lichtweg (34) verläuft, und
- ein Transportrad (10), mit dem die Probe (60) in den Lichtweg (34) transportierbar ist, wobei
- der Lichtweg (34) auf eine Transportkammer (13) des Transportrads (10) gerichtet ist,
- das Transportrad (10) in einem Kammergehäuse (20) angeordnet ist, und wobei
- in mindestens einer Wand des Kammergehäuses (20) ein transparentes Fenster (24) vorgesehen ist, durch das der Lichtweg (34) verläuft,
**dadurch gekennzeichnet, dass**
- das transparente Fenster (24) durch einen optischen Filter gebildet wird, wobei
- die Messeinrichtung mit einer Stelleinrichtung (27) zur Betätigung des Transportrads (10) ausgestattet ist und die Stelleinrichtung zur diskontinuierlichen Betätigung mit verschiedenen Richtungen der Förderbewegung des Transportrads (10) eingerichtet ist, so dass eine Verdichtung der Probe (60) in der Transportkammer (13) erreichbar ist.

12. Messeinrichtung nach Anspruch 11, bei der das Transportrad (10) ein Schaufelrad (12) mit einer oder mehreren Transportkammern (13) umfasst.

13. Messeinrichtung nach mindestens einem der Ansprüche 11 oder 12, bei der die Detektoreinrichtung (40) dazu eingerichtet ist, eine Messung an der Probe (60) in der optischen Messstrecke (34) durchzuführen, wenn das Transportrad (10) nicht betätigt wird und die Probe (60) im Lichtweg (34) ruht.

14. Messeinrichtung nach mindestens einem der Ansprüche 11 bis 13, bei der der optische Filter aus einem Material gebildet ist, das unempfindlich gegen elektrostatische Aufladungen ist.

15. Messeinrichtung nach mindestens einem der Ansprüche 11 bis 14, bei der die Länge des durch die Transportkammer (13) verlaufenden Teils des Lichtwegs (34) veränderlich ist.

16. Messeinrichtung nach mindestens einem der Ansprüche 11 bis 15, bei der die Detektoreinrichtung (40) zur Messung der Transmission oder optischen Dichte der Probe (60) im Lichtweg (34) eingerichtet ist.

17. Messeinrichtung nach mindestens einem der Ansprüche 11 bis 15, bei der die Detektoreinrichtung (40) zur Messung der Reflektion der Probe (60) im Lichtweg (34) eingerichtet ist.

18. Messeinrichtung nach Anspruch 17, bei der die Detektoreinrichtung (40) eine Integrationskugel (41) aufweist.

19. Getreidefördereinrichtung, die mit einer Messeinrichtung nach mindestens einem der Ansprüche 11 bis 18 ausgestattet ist.

20. Getreidefördereinrichtung nach Anspruch 19, die mit einer Getreide-Transportleitung verbunden ist.

## Claims

1. Method for measuring at least one spectroscopic parameter of a sample (60), which comprises a plurality of particles,
• in which the sample (60) is transported by a transport wheel (10) into a light path (34) of an illumination device (30) and
• there undergoes measurement of the at least one spectroscopic parameter, wherein
• the measurement of the at least one spectroscopic parameter occurs while the sample (60) is located in a transport chamber (13) of the transport wheel (10),
• the light path (34) is directed onto the transport chamber (13),
• the transport wheel (10) is arranged in a chamber housing (20), and wherein
• in at least one wall of the chamber housing (20) a transparent window (24) is provided, through which the light path (34) runs,
**characterised in that**
• the transparent window (24) is formed by an optical filter and
• the transport wheel (10) is operated discontinuously, wherein transport movements of the transport wheel (10) change with different directions, so that the sample (60) is compacted in the transport chamber (13).

2. Method according to claim 1, in which the detection of the at least one spectroscopic parameter occurs while the transport wheel (10) is not moving and the sample (50) is resting in relation to the light path (34).

3. Method according to claim 2, in which a compaction of the sample (60) occurs before detection of the at least one spectroscopic parameter.

4. Method according to at least one of the preceding claims, in which the transmission, optical density and/or reflection of the sample (50) located in the measurement path (34) is detected as spectroscopic parameter.

5. Method according to claim 4, in which a difference value and/or spectrum formation occurs.

6. Method according to at least one of the preceding claims, in which a layer thickness of the sample (60) is adjusted along the light path (34) by expanding or diminishing the transport chamber (13).

7. Method according to at least one of the preceding claims, in which a bucket wheel (10) with one or more transport chambers (13) is used as transport wheel.

8. Method according to at least one of the preceding claims, in which the light path (34) runs parallel to the rotational axis of the bucket wheel (12).

9. Method according to at least one of the preceding claims, in which the sample (60) comprises agricultural products in particle form or synthetic particles.

10. Method according to claim 9, in which the sample (60) comprises grain.

11. Measuring device (100) for measuring spectroscopic properties of a sample (60) with a plurality of particles, comprising:
• an illumination device (30) and a detector device (40), between which a light path (34) runs, and
• a transport wheel (10), with which the sample (60) can be transported into the light path (34), wherein
• the light path (34) is directed onto a transport chamber (13) of the transport wheel (10),
• the transport wheel (10) is arranged in a chamber housing (20), and wherein
• in at least one wall of the chamber housing (20) a transparent window (24) is provided, through which the light path (34) runs,
**characterised in that**
• the transparent window (24) is formed by an optical filter, wherein
• the measurement device is equipped with an actuating means (27) for operating the transport wheel (10) and the actuating means is fitted for discontinuous operation with different directions of the transport movement of the transport wheel (10), so that a compaction of the sample (60) can be achieved in the transport chamber (13).

12. Measuring device according to claim 11, in which the transport wheel (10) comprises a bucket wheel (12) with one or more transport chambers (13).

13. Measuring device according to at least one of claims 11 or 12, in which the detector device (40) is fitted to conduct a measurement on the sample (60) in the optical measurement path (34) when the transport wheel (10) is not operated and the sample (60) is resting in the light path (34).

14. Measuring device according to at least one of claims 11 to 13, in which the optical filter is formed from a material that is insensitive to electrostatic charges.

15. Measuring device according to at least one of claims 11 to 14, in which the length of the part of the light path (34) running through the transport chamber (13) is variable.

16. Measuring device according to at least one of claims 11 to 15, in which the detector device (40) is fitted for measurement of the transmission or optical density of the sample (60) in the light path (34).

17. Measuring device according to at least one of claims 11 to 15, in which the detector device (40) is fitted for measurement of the reflection of the sample (60) in the light path (34).

18. Measuring device according to claim 17, in which the detector device (40) has an integrating sphere (41).

19. Grain transport assembly, which is equipped with a measuring device according to at least one of claims 11 to 18.

20. Grain transport assembly according to claim 19, which is connected to a grain transport tube.

## Revendications

1. Procédé pour la mesure d'au moins un paramètre spectroscopique d'un échantillon (60) qui comprend une pluralité de particules,
- dans lequel l'échantillon (60) est transporté avec une roue de transport (10) dans un trajet de faisceau lumineux (34) d'un dispositif d'éclairage (30) et
- là, il est soumis à la mesure du au moins un paramètre spectroscopique,
- la mesure du au moins un paramètre spectroscopique se faisant pendant que l'échantillon (60) se trouve dans une chambre de transport (13) de la roue de transport (10),
- le trajet de faisceau lumineux (34) étant dirigé vers la chambre de transport (13),
- la roue de transport (10) étant disposée dans un boîtier de chambre (20), et
- dans au moins une paroi du boîtier de chambre (20), il est prévu une fenêtre transparente (24) à travers laquelle passe le trajet de faisceau lumineux (34),
**caractérisé en ce que**
- la fenêtre transparente (24) est formée par un filtre optique et
- la roue de transport (10) fonctionne de manière discontinue, les mouvements de transport de la roue de transport (10) alternant avec des directions différentes, de sorte que l'échantillon (60) est compressé dans la chambre de transport (13).

2. Procédé selon la revendication 1, dans lequel la détection du au moins un paramètre spectroscopique se fait pendant que la roue de transport (10) ne se déplace pas et que l'échantillon (50) est au repos par rapport au trajet de faisceau lumineux (34).

3. Procédé selon la revendication 2, dans lequel une compression de l'échantillon (60) est réalisée avant la détection du au moins un paramètre spectroscopique.

4. Procédé selon au moins l'une quelconque des revendications précédentes, dans lequel la transmission, la densité optique et/ou la réflexion de l'échantillon (50) se trouvant dans le trajet de faisceau lumineux (34) est détectée comme paramètre spectroscopique.

5. Procédé selon la revendication 4, dans lequel une valeur différentielle et/ou un spectre différentiel est formé(e).

6. Procédé selon au moins l'une quelconque des revendications précédentes, dans lequel une épaisseur de couche de l'échantillon (60) est réglée le long du trajet de faisceau lumineux (34) par un agrandissement ou une réduction de la chambre de transport (13).

7. Procédé selon au moins l'une quelconque des revendications précédentes, dans lequel une roue à palettes avec une ou plusieurs chambres de transport (13) est utilisée comme roue de transport (10).

8. Procédé selon au moins l'une quelconque des revendications précédentes, dans lequel le trajet de faisceau lumineux (34) s'étend parallèlement à l'axe de rotation de la roue à palettes (12).

9. Procédé selon au moins l'une quelconque des revendications précédentes, dans lequel l'échantillon (60) comprend des produits agricoles particulaires ou des particules synthétiques.

10. Procédé selon la revendication 9, dans lequel l'échantillon (60) comprend des céréales.

11. Dispositif de mesure (100) pour la mesure des caractéristiques spectroscopiques d'un échantillon (60) avec une pluralité de particules, comprenant :
- un dispositif d'éclairage (30) et un dispositif de détection (40) entre lesquels s'étend un trajet de faisceau lumineux (34), et
- une roue de transport (10) avec laquelle l'échantillon (60) peut être transporté dans le trajet de faisceau lumineux (34) ,
- le trajet de faisceau lumineux (34) étant dirigé vers une chambre de transport (13) de la roue de transport (10),
- la roue de transport (10) étant disposée dans un boîtier de chambre (20), et
- dans au moins une paroi du boîtier de chambre (20), il est prévu une fenêtre transparente (24) à travers laquelle passe le trajet de faisceau lumineux (34),
**caractérisé en ce que**
- la fenêtre transparente (24) est formée par un filtre optique,
- le dispositif de mesure étant équipé d'un dispositif de réglage (27) pour actionner la roue de transport (10) et le dispositif de réglage étant conçu pour le fonctionnement discontinu avec des directions différentes du mouvement de transport de la roue de transport (10), de sorte que l'on peut obtenir une compression de l'échantillon (60) dans la chambre de transport (13).

12. Dispositif de mesure selon la revendication 11, dans lequel la roue de transport (10) comprend une roue à palettes (12) avec une ou plusieurs chambres de transport (13).

13. Dispositif de mesure selon au moins l'une quelconque des revendications 11 ou 12, dans lequel le dispositif de détection (40) est conçu pour effectuer une mesure sur l'échantillon (60) dans le trajet de faisceau optique (34) lorsque la roue de transport (10) n'est pas actionnée et que l'échantillon (60) est au repos dans le trajet de faisceau lumineux (34).

14. Dispositif de mesure selon au moins l'une quelconque des revendications 11 à 13, dans lequel le filtre optique est constitué d'un matériau qui est insensible aux charges électrostatiques.

15. Dispositif de mesure selon au moins l'une quelconque des revendications 11 à 14, dans lequel la longueur de la partie du trajet de faisceau lumineux (34) passant à travers la chambre de transport (13) est variable.

16. Dispositif de mesure selon au moins l'une quelconque des revendications 11 à 15, dans lequel le dispositif de détection (40) est conçu pour la mesure de la transmission ou de la densité optique de l'échantillon (60) dans le trajet de faisceau lumineux (34).

17. Dispositif de mesure selon au moins l'une quelconque des revendications 11 à 15, dans lequel le dispositif de détection (40) est conçu pour la mesure de la réflexion de l'échantillon (60) dans le trajet de faisceau lumineux (34).

18. Dispositif de mesure selon la revendication 17, dans lequel le dispositif de détection (40) comporte une bille d'intégration (41).

19. Dispositif de transport de céréales qui est équipé d'un dispositif de mesure selon au moins l'une quelconque des revendications 11 à 18.

20. Dispositif de transport de céréales selon la revendication 19, qui est raccordé à une conduite de transport de céréales.
